# EUROPÄISCHE PATENTSCHRIFT

(11) **EP 1 285 446 B1**
(45) Veröffentlichungstag und Bekanntmachung des Hinweises auf die Patenterteilung: **02.11.2005**
(21) Anmeldenummer: 02730004.5
(22) Anmeldetag: 13.03.2002
(51) Int. Cl.: H01B 7/04, A61B 5/0428, H01B 7/00

(54) **ÜBERTRAGUNGSKABEL FÜR MEDIZINISCHE SIGNALWERTE**
TRANSMISSION CABLE FOR MEDICAL SIGNAL VALUES
CABLE DE TRANSMISSION POUR VALEURS DE SIGNALISATION MEDICALES

(30) Priorität: 14.03.2001 DE 10112051
(43) Veröffentlichungstag der Anmeldung: 26.02.2003
(73) Patentinhaber: Leoni Kabel GmbH & Co KG, 90402 Nürnberg (DE)
(72) Erfinder: WECHSLER, Peter, 91180 Heideck (DE)
(74) Vertreter: Tergau & Pohl Patentanwälte
(86) Internationale Anmeldenummer: PCT/EP2002/002773
(87) Internationale Veröffentlichungsnummer: WO 2002/086915

(56) Entgegenhaltungen:
- EP-A- 0 553 372
- EP-A- 0 685 239
- US-B1- 6 171 255

## Beschreibung

Die Erfindung bezieht sich auf ein Übertragungskabel für medizinische Signalwerte. Hierunter werden insbesondere Elektrokardiogramm (EKG)-, Temperatur-, Stickstoff- und Blutdruck-Werte verstanden.

Zur Übertragung derartiger Signalwerte werden in der Medizintechnik üblicherweise unterschiedliche Kabeltypen eingesetzt. So werden beispielsweise zur Übertragung von EKG-, Temperatur- und Stickstoffwerten üblicherweise elektrische Leiter mit einer metallischen Leiterader und einer diese umgebenden Leiterisolierung eingesetzt, während zur Übertragung von Blutdruckwerten flexible Schläuche als Hohlleiter verwendet werden. Dies führt in unerwünschter Weise zu einer aufwendigen Verkabelung bei der Übertragung von Signalwerten zu medizinischen Zwecken.

Aus der EP 0 685 239 A2 ist ein Kabel zur Übertragung von medizinischen Signalwerten bekannt, bei dem in einem zentralen Bereich drei Einzelleitungen angeordnet sind, die Signalwerte für eine Blutsauerstoffmessung führen. Dieser zentrale Bereich ist umgeben von einer Abschirmung. In einem Ringraum um diese Abschirmung sind weitere Einzelleitungen angeordnet, die insbesondere Signalwerte einer Temperaturmessung, EKG-Werte bzw. weitere Sigalwerte für die Blutsauerstoffmessung führen. In der EP 0 685 239 A2 ist ferner offenbart, dass die besagte Abschirmung zwischen dem zentralen Bereich und dem diesen umgebenden Ringraum als Luftschlauch eines nicht-invasiven Blutdruckmessgerätes ausgebildet sein kann.

Aus der US 6,171,255 B1 ist ein weiteres Kabel zur Übertragung von medizinischen Signalwerten bekannt, bei dem ein Luftschlauch eines Blutdruckmessgeräts neben einem elektrischen Einzelleiter zur Übertragung von Druckwerten und einem weiteren elektrischen Einzelleiter zur Übertragung von Signalwerten eines Sensors geführt ist.

Der Erfindung liegt die Aufgabe zugrunde, die Verkabelung zur Übertragung von medizinischen Signalwerten möglichst weitgehend zu vereinfachen.

Diese Aufgabe wird erfindungsgemäß gelöst durch die Merkmale des Anspruchs 1. Dazu sind mehrere Einzelkabel unterschiedlichen Kabeltyps von einem gemeinsamen Kabelmantel umgeben und dabei derart in diesen integriert, dass ein einziges, praktisch multifunktionales Übertragungskabel für medizinische Signalwerte bereitgestellt ist, wobei jeder Kabeltyp zur Übertragung eines speziellen medizinischen Signalwertes ausgebildet ist. Der Aufbau des Übertragungskabels ist dabei zweckmäßigerweise symmetrisch, wobei die Einzelkabel in einem am Umfang eines zentralen, speziellen Einzelkabels gebildeten Ringraum verteilt angeordnet sind.

Zweckmäßigerweise sind je Kabeltyp mehrere Einzelkabel zur Übertragung des jeweils speziellen medizinischen Signalwertes innerhalb des Übertragungskabel vorgesehen . So können beispielsweise zur Übertragung von Temperaturwerten zwei oder drei Einzelkabel gleichen Kabeltyps vorgesehen sein. Ebenso können zur Übertragung von Elektrokardiogramm-Werten zwei, drei oder auch mehr, z. B. sechs, Einzelkabel gleichen Typs vorgesehen sein. Zur Übertragung von Werten des Stickstoffgehaltes im Blut können zwei bis fünf Einzelkabel vorgesehen sein. Dabei können zur Übertragung dieser Signalwerte wiederum unterschiedliche Kabeltypen, insbesondere zusätzlich oder alternativ aus miteinander verseilten symmetrischen oder koaxialen Kabeln aufgebaute Einzelkabel, vorgesehen sein.

Zur Übertragung von Blutdruckwerten, d.h. sich aus Blutdruckmessungen ergebenden Signalwerten, ist ein flexibler, nachfolgend auch als Hohlleiter bezeichneter Schlauch vorgesehen, der innerhalb des Übertragungskabels zentral angeordnet ist An dessen Außenumfang sind dann die übrigen Einzelkabel verteilt angeordnet. Dabei liegen diese übrigen Einzelkabel zweckmäßigerweise in einem Ringraum zwischen dem Schlauch und dem allen Einzelkabeln gemeinsamen Außen- oder Kabelmantel des Übertragungskabels. Dabei können alle Einzelkabel zweckmäßigerweise mittels einer z. B. geflochtenen Abschirmung umgeben sein.

Zur Vermeidung von Lücken oder Zwischenräumen im Ringraum können je nach Anzahl der den Schlauch umgebenden Einzelkabel eine entsprechende Anzahl von Füllelementen vorgesehen sein, so dass der Ringraum vollständig ausgefüllt ist. Dies wiederum führt zu einem räumlich homogenen und damit besonders vorteilhaften symmetrischen Aufbau des Übertragungskabels. Dabei weisen sowohl die im Ringraum angeordneten Einzelkabel als auch die Füllelemente jeweils zumindest annähernd den gleichen Außendurchmesser auf, um eine möglichst symmetrische und stabile Gesamtkonfiguration sowie eine gleichmäßige Außenkontur des Übertragungskabels zu erreichen. Auch kann zwischen dem Ringraum und dem Schlauch eine Bandierung, z.B. ein Vlies, zur Erhöhung der Zugfestigkeit des Übertragungskabels vorgesehen sein.

Die mit der Erfindung erzielten Vorteile bestehen insbesondere darin, dass durch die Integration mehrere Einzelkabel unterschiedlichen Kabeltyps zur Übertragung spezieller medizinischer Signalwerte in einem gemeinsamen Kabelmantel ein einziges multifunktionales Übertragungskabel oder Multifunktionskabel für medizinische Zwecke bereitgestellt ist, in dem insbesondere die Funktionswerte EKG, Temperatur, Stickstoffgehalt im Blut und Blutdruckmessung zusammengefasst sind. Da somit vorzugsweise zumindest diese medizinischen Signalwerte mit diesem einzelnen Multifunktionskabel übertragbar sind, ist der Verkabelungsaufwand praktisch minimiert.

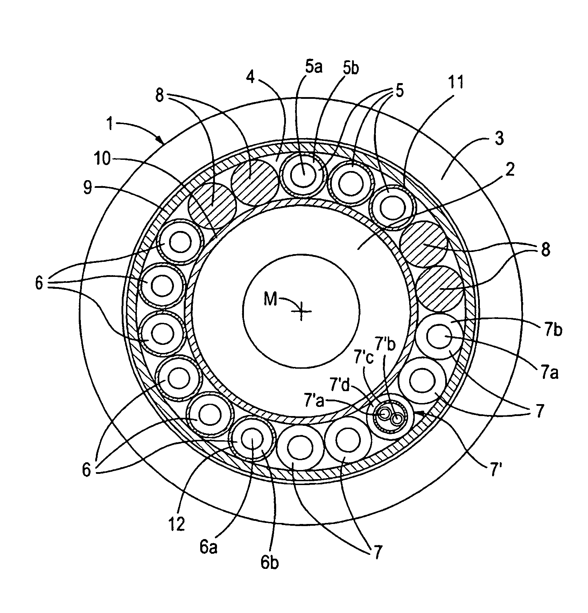

Nachfolgend wird ein Ausführungsbeispiel der Erfindung anhand einer Zeichnung näher erläutert. Darin zeigt die Figur im Querschnitt ein erfindungsgemäßes Übertragungskabel für medizinische Zwecke mit einer Anzahl von Einzelkabeln unterschiedlichen Kabeltyps.

Das nachfolgend als Multifunktionskabel bezeichnete Übertragungskabel 1 zur Übertragung unterschiedlicher medizinischer Signalwerte umfasst einen Hohlleiter oder Schlauch 2, der koaxial um die Kabel- oder Mittellängsachse M und somit zentral angeordnet ist. Zwischen dem Schlauch 2 als spezielles Einzelkabel zur Übertragung von Blutdruckwerten und einem Kabelmantel oder Kabelaußenmantel 3 ist ein Ringraum 4 gebildet, in dem eine Vielzahl von Einzelkabeln 5,6,7,7' unterschiedlichen Kabeltyps angeordnet sind. Der Kabelmantel 3 besteht vorteilhafterweise aus einem thermoplastischen Kunststoff, z. B. auf Urethanbasis.

So sind eine erste Anzahl von Einzelkabeln 5, im Ausführungsbeispiel drei Einzelkabel 5, zur Übertragung von Temperaturwerten im Ringraum 4 angeordnet. Eine weitere Anzahl von Einzeikabeln 6, im Ausführungsbeispiel sechs Einzelkabel 6, sind zur Übertragung von Elektrokardiogramm- oder EKG-Werten vorgesehen. Weitere Einzelkabel 7,7' dienen zur Übertragung von Signalwerten des Stickstoffgehaltes im Blut.

Im Ringraum 4 sind je nach Anzahl der Einzelkabel 5,6,7,7' unterschiedlichen Kabeltyps eine zur Auffüllung des Ringraums 4 dienende Anzahl von Füllelementen 8, im Ausführungsbeispiel vier Füllelemente 8, vorgesehen. Zwischen dem Ringraum 4 und dem Kabelmantel 3 ist eine Abschirmung 9 vorgesehen, die den Ringraum und somit die Einzelkabel 4 bis 7 und 7' gegen den Kabelmantel 3 und damit gegen die Umgebung abschirmt. Die Abschirmung 9 kann zweckmäßigerweise ein Drahtgeflecht sein. Zwischen dem Ringraum 4 und dem Hohlleiter 2 ist eine Bandierung 10, vorzugsweise in Form eines Vlieses, vorgesehen.

Die Außendurchmesser der Einzelkabel 5,6,7 sowie der Füllelemente 8 sind aneinander angepasst, so dass die Breite des Ringraums 4 über den gesamten Umfang des Schlauches 2 zumindest annähernd gleich ist. Somit ergibt sich ein um die Mittelachse M symmetrisches Übertragungs- oder Multifunktionskabel 1. Hierzu sind insbesondere die Einzelkabel 5 und 6 mit einem entsprechenden Außenmantel 11 bzw. 12 ummantelt, um einen den Einzelkabeln 7 und den Füllelementen 8 entsprechenden Außendurchmesser zu gewährleisten.

### Bezugszeichenliste

- 1: Übertragungskabel
- 2: Schlauch/Hohlleiter

- M: Mittelachse

- 3: Kabel-/Außenmantel
- 4: Ringraum
- 5: Einzelkabel
- 6: Einzelkabel
- 7: Einzelkabel
- 8: Füllelement
- 9: Abschirmung
- 10: Bandierung
- 11,12: Außenmantel

## Patentansprüche

1. Übertragungskabel (1) für medizinische Signalwerte, mit mehreren in einem gemeinsamen Kabelmantel (3) integrierten Einzelkabeln (2,5,6,7,7') unterschiedlichen Kabeltyps, wobei jeder Kabeltyp zur Übertragung eines speziellen medizinischen Signalwertes ausgebildet ist,
- wobei eines der Einzelkabel als flexibler Schlauch (2) zur Übertragung von Blutdruckwerten ausgebildet ist,
- wobei eine erste Anzahl von Einzelkabeln (7,7') für Werte des Stickstoffgehaltes im Blut, eine zweite Anzahl von Einzelkabeln (5) für Temperaturwerte und eine dritte Anzahl von Einzelkabeln (6) für EKG-Werte vorgesehen sind,
**gekennzeichnet dadurch, dass**
der Schlauch (2) zentral angeordnet ist und alle übrigen Einzelkabel (5,6,7,7') in einem Ringraum (4) zwischen dem Schlauch (2) und dem Kabelmantel (3) angeordnet sind und
zwischen dem Ringraum (4) und dem Kabelmantel (3) eine Abschirmung (9) vorgesehen ist.

2. Übertragungskabel nach Anspruch 1, wobei zwischen dem Ringraum (4) und dem Schlauch (2) eine Bandierung (10) vorgesehen ist.

3. Übertragungskabel nach Anspruch 1 oder 2, wobei in dem Ringraum (4) eine Anzahl von Füllelementen (8) angeordnet sind.

4. Übertragungskabel nach einem der Ansprüche 1 bis 3, wobei als Isolationsmaterial für den Kabelmantel (3) ein thermoplastischer Kunststoff verwendet ist.

## Claims

1. Transmission cable (1) for medical signal values, having a number of individual cables (2, 5, 6, 7, 7') of different cable type which are integrated in a common cable sheath (3), each cable type being designed for transmitting a specific medical signal value,
- one of the individual cables being designed as a flexible tube (2) for transmitting blood pressure values, and
- a first number of individual cables (7, 7') being provided for values of the nitrogen content in the blood, a second number of individual cables (5) being provided for temperature values, and a third number of individual cables (6) being provided for ECG values,
**characterized in that** the tube (2) is arranged centrally and all the remaining individual cables (5, 6, 7, 7') are arranged in an annular space (4) between the tube (2) and the cable sheath (3), and a screen (9) is provided between the annular space (4) and the cable sheath (3).

2. Transmission cable according to Claim 1, in which a banding (10) is provided between the annular space (4) and the tube (2).

3. Transmission cable according to Claim 1 or 2, in which a number of filling elements (8) are arranged in the annular space (4).

4. Transmission cable according to one of Claims 1 to 3, in which a thermoplastic material is used as insulation material for the cable sheath (3).

## Revendications

1. Câble de transmission pour des valeurs de signaux médicaux, comportant plusieurs câbles individuels (2, 5, 6, 7, 7') de différents types, intégrés dans une gaine commune (3) pour câbles, chaque type de câble étant conçu pour la transmission d'une valeur médical spécial de signal
- dans lequel l'un des câbles individuels est agencé sous la forme d'un tuyau flexible (2) pour la transmission de valeurs de la pression sanguine,
- dans lequel un premier nombre de câbles individuels (7, 7') sont prévus pour des valeurs de la teneur en oxygène dans le sang, un second nombre de câbles individuels (5) sont prévus pour des valeurs de température et un troisième nombre de câbles individuels (6) sont prévus pour les valeurs d'électrocardiogramme,
**caractérisé en ce que**
le tuyau (2) est disposé d'une manière centrale et tous les autres câbles individuels (5, 6 , 7, 7') sont disposés dans une chambre annulaire (4) présente entre le tuyau (2) et la gaine (3) du câble, et qu'un blindage (9) est prévu entre la chambre annulaire (4) et la gaine (3) du câble.

2. Câble de transmission selon la revendication 1, dans lequel un bandage (10) est prévu entre la chambre annulaire (7) et le tuyau (2).

3. Câble de transmission selon la revendication 1 ou 2, dans lequel un nombre d'éléments de remplissage (8) sont disposés dans la chambre annulaire (4).

4. Câble de transmission selon l'une des revendications 1 à 3, dans lequel on utilise une matière thermoplastique en tant que matériau d'isolation pour la gaine (3) du câble.
